# EUROPEAN PATENT SPECIFICATION

(11) **EP 2 131 779 B1**
(45) Date of publication and mention of the grant of the patent: **06.01.2016**
(21) Application number: 08735710.9
(22) Date of filing: 02.04.2008
(51) Int. Cl.: A61C 1/08, A61C 8/00, A61B 17/16

(54) **A SURGICAL BONE MILLING INSTRUMENT**
CHIRURGISCHER KNOCHENFRÄSER
INSTRUMENT CHIRURGICAL UTILISÉ POUR LE FRAISAGE DES OS

(30) Priority: 12.04.2007 IT RE20070054
(43) Date of publication of application: 16.12.2009
(73) Proprietor: C.G.M. S.P.A., 42015 Correggio (Reggio Emilia) (IT)
(72) Inventor: PARMIGIANI, Corrado, Saverio, 42015 Correggio (Reggio Emilia) (IT)
(74) Representative: Corradini, Corrado
(86) International application number: PCT/EP2008/053961
(87) International publication number: WO 2008/125502

(56) References cited:
- US-A- 5 885 293
- US-A- 6 096 038
- US-A1- 2002 022 847
- US-A1- 2002 032 447

## Description

### Technical Field

The present invention relates to a surgical bone milling instrument.

### Background Art

In general, the purpose of the instrument is the removal of areas of bone in a controlled manner in proximity to delicate parts of bone, typically to complete holes in bones in which the a terminal portion of the hole is located close to particularly delicate organs.

A typical application is in the surgical technique of mini-lifting of the maxillary sinus cavity, which involves the raising of the floor and filling with biomaterial of the maxillary cavity through a hole prepared in the bone for the insertion of a dental implant.

The cavity in the maxillary sinus is the largest of the pneumatic cavities in the cranial bone and it is located in the rear areas above the upper jaw.

The formation of the maxillary sinus floor can be influenced by the presence of the roots of premolar and molar teeth. At the apex of these roots there is a thin layer of compact cortical bone (maxillary sinus floor). The maxillary sinus cavity is normally unitary and contains air. The inside of the cavity is coated entirely with a mucus membrane known as the Schneider membrane.

The insertion of dental implants in the rear areas of the upper jaw is almost always conditioned by the presence of the maxillary sinus, which limits the availability of bone in height, especially in patients who have been edentulous for a long time.

Following the loss of the rear teeth of the jaw, a process of reabsorption of "external" bone begins, combined with a like reabsorption of the floor of the "internal" maxillary sinus, causing expansion of the maxillary sinus cavity and the approach of the sinus floor progressively closer to the "outside" edge of the alveolar crest, reducing the height of bone available for implantation.

In the 1990s the above-mentioned surgical techniques for the mini-lifting of the maxillary sinus were developed, substantially involving the boring of holes in the jaw for the insertion of dental implants and the raising of the maxillary sinus floor and its filling with biomaterial of the maxillary cavity through the hole.

These techniques penetrate to the Schneider membrane, depending only on tactile sensation and radiographic investigations for its identification, since the membrane is not directly but only indirectly visible.

These techniques attracted the interest of dental surgeons less familiar with advanced surgery. The maxillary sinus mini-lifting technique offers functional advantages in view of the incidence of the operation.

The main difficulty of these techniques for raising the maxillary sinus is the creation of the final part of the bone cavity with decollement/detachment of the Schneider membrane from the "internal" bone surface in order to permit the filling of the sinus cavity with bone tissue (biomaterial). The limited thickness of the membrane or the slightest error of the operator can result in lacerations that compromise the success of the filling of the sinus cavity. The current techniques for mini-lifting can be divided on the basis of the instruments used, which are either osteotomes or mills.

Osteotomes are manual instruments, increasing in size and provided with a concave point with a cutting edge. Osteotomes can be used with depth limit stop rings adjusted manually with fixing screws. The stop is positioned according to the available bone height, established radiographically. An osteotome can be used with manual pressure or, in the case of hard bone, with the help of a surgical mallet. The special conformation of the osteotome means that once it is inserted into the implant hole, prepared to a distance of 1 to 2 mm from the floor of the maxillary sinus, it can remove a small quantity of bone tissue from the walls of the site and concentrate this on the terminal section of the osteotome. The bone cortex of the floor of the maxillary sinus is then fractured, by way of percussion with a mallet, to raise the floor of the sinus until the required lifting is achieved.

This operation requires extreme delicacy to avoid the possibility of lacerating the thin Schneider membrane.

In order to reduce the risk of laceration of the membrane, instead of using osteotomes alone, the insertion of biomaterial into the bone cavity has been proposed in order to act as padding to be compacted vertically between the osteotome point and the bone.

Mini-lifting mills are rotating instruments (500 rpm) for fitting on an electrical turbine and are fitted with a non cutting point and available in various calibrated lengths (one mill every millimetre) for sequential use. The mills act by wearing away the bone cortex that precedes the floor of the maxillary sinus, and because they are not sharp they can be used on the final portion of bone, limiting the risk of damage to the Schneider membrane.

After preparation of the site and after decollement and raising of the membrane using rounded manual instruments, the biomaterial is inserted before the positioning of the dental insert.

This technique again depends on the tactile sensitivity and practical experience of the operator.

One aim of the present invention is to realize a device capable of overcoming the difficulties mentioned above.

These and other aims are achieved by the invention as it is characterized in the accompanying claims.

### Apparatuses for creating cavities in bone are known for example from US 2002/0032447 and US 6096038. Disclosure of Invention

The invention is as claimed in claim 1. The invention makes it possible to complete axial milling of a bone hole (formed previously using a standard instrument) maintaining control of the position of the device relative to the hole. The action of the probe element of the instrument enables the operator to perceive, also visually, the moment when the milling head of the instrument reaches the end of the previously formed hole, or the end of the excavation created by the milling head.

From this point onwards the operations of further axial advance of the milling head can be monitored with the probe element; while the milling element is further advanced, to excavate an additional axial section of groove, the probe device remains axially stationary against the central area of the end surface (the part not subjected to milling). Putting millimetric markings on the probe element makes it a means for continuous control and determination of the axial position of the milling head relative to the position of the end of the bone hole.

Furthermore, by way of axial pressure applied to the probe element the detachment of the residual portion of the bone wall can be achieved as soon as the residual portion has reached a breaking resistance level that is lower than the force applied to the probe element.

### Brief description of the Drawings

The invention will be described in detail below with the aid of the attached figures of the drawings, which illustrate a non-exclusive embodiment of the invention, by way of example, and in which:
Figure 1 is a side view of a first embodiment of the instrument of the invention.
Figure 2 is a cross-section along the axial plane II-II of figure 1.
Figures 2A and 2B show the milling head 11 of figure 2, in enlarged scale, respectively in the advanced and withdrawn positions.
Figure 3 is a perspective view of figure 1.
Figures 4A and 4B show an enlarged detail of figure 1 in two different operating positions.
Figure 5 is the cross-section along the transverse plane V-V of figure 4A.
Figures from 6A to 6F show a sequence of operating stages in which the instrument excavates the final section of a hole in a bone.
Figure 7 is an exploded view of the instrument of Figure 1.
Figure 8 is an axial cross-section of a second embodiment of the instrument of the invention.

### Best Mode for Currying Out the Invention

The instrument 1 illustrated in figures from 1 to 5 comprises a milling element 10, in particular, having an elongate body shape with a longitudinal axis A, the forward portion of which can rotate about the axis A and mill the bone. In particular, the extreme forward portion of the milling device 10 forms a milling head 11 which is equipped with sharp raised milling ridges 12, capable of milling bone in an axial direction when rotating.

The instrument 1 comprises a probe element 20, in particular, with an elongate shape located internally of and coaxial with the milling element 10 and sliding longitudinally, along axis A, through the milling element 10, with a forward portion 21 projecting beyond the forward portion of the milling element 10. In particular, the probe element 20 is inserted into an axial through-hole 15 formed in the milling element 10; the raised milling ridges 12 thus have a radial extension limited by the presence of the hole 15 in the head 11.

In particular, the probe element 20, at least in its forward and mid portions, is shaped as a cylindrical rod, possibly with the forward portion 21 rounded in order not to harm the tissues with which it comes into contact.

The probe element 20 is capable of indicating to the operator, also visually, when the milling head 11 reaches the end of the hole into which it is inserted during the use of the instrument and, from that point onwards, the position of the milling head 11 in the subsequent operations of further axial advance of the milling head 11.

Further, by way of an axial pressure applied to the probe element 20, it is possible to cause the detachment of the residual portion of bone wall, when the bone wall has reached a resistance to breakage which is lower than the thrust force applied to the probe element 20.

The instrument 1 comprises a screw-threaded tubular element 30 through which the milling element 10 is inserted coaxially, with the milling head 11 thereof projecting beyond the end of the tubular element 30. In particular the tubular element 30 has a cylindrical tubular body, an outside surface of which has a circular cross-section and is provided with a thread 31 a, which engages by helical coupling in the hole formed in the bone.

The milling element 10 is associated to the tubular element 30 and can rotate around the longitudinal axis A relative to the tubular element 30, and can displace axially with respect thereto.

In particular, the milling element 10 has a rear portion which coaxially passes through the tubular element; in particular, this comprises a tubular rod 14 of constant cross-section located behind the milling head 11, the external cylindrical surface of which snugly couples with and matches the size of the internal cylindrical surface 32 of the threaded tubular element 30, the length of the rod 14 being greater than that of the tubular element 30. The head 11 has a greater external diameter than the rod 14 and its rear portion has a radial rear edge 11 b designed to stop in striking contact with the circular edge of the front end 30a of the tubular element 30. The external diameter of the head 11 is approximately equal to the external diameter of the tubular element 30, in particular, it is smaller than the external diameter of the thread 31 a.

The milling device 10 is solidly fixed to a drive element 40, located coaxially and to the rear of the tubular element 30, causing the rotation of the milling element and translating it axially in relation to the tubular element 30. In particular, the drive element 40 is coaxially and solidly joined to the rear portion of the rod 14; the drive element 40 has a forward shank 41, of lesser diameter, and a rear portion 42, of greater diameter, shaped as a circular handle for enabling manual activation of the milling element 10, in rotation and in axial translation. The rear portion 42 also serves as a handle for the manipulation of the entire instrument 1.

In the embodiment shown in the figures, the drive element 40 also acts to transmit torque to the tubular element 30, causing it to rotate.

In particular, the drive element 40, is free to slide axially, over a certain distance, relative to the tubular element 30, and to rotate, again relative to the tubular element 30, and is torsionally engaged to the tubular element 30 by reciprocal engaging means that leave the drive element 40 free to be rotated by an angle of less than 360 degrees and to be axially displaced in relation to the tubular element 30.

In particular, the reciprocal engaging means comprise profiled raised portions 35 and 45 extending in an axial direction towards each other (one faces back and the other forward), from the extreme rear end circular edge 30b of the tubular element 30 and respectively from the extreme forward end circular edge 42a of the shank 42 of the drive element, the raised portions of which are designed to reciprocally strikingly contact following reciprocal rotation in order to transmit torque.

The instrument comprises means for limiting the axial displacement of the milling element 10, together with the drive element 40, with respect to the tubular element 30.

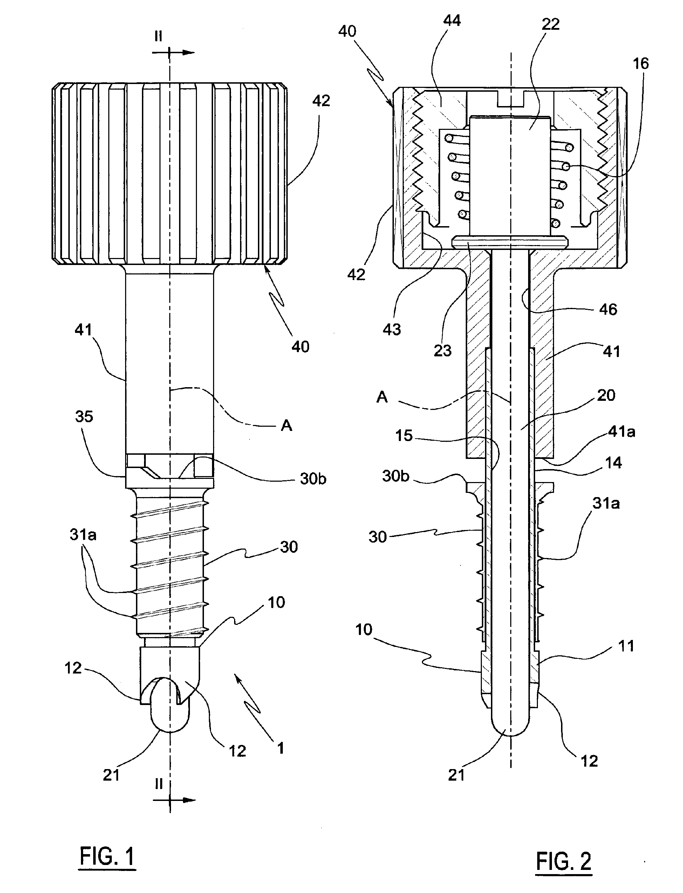

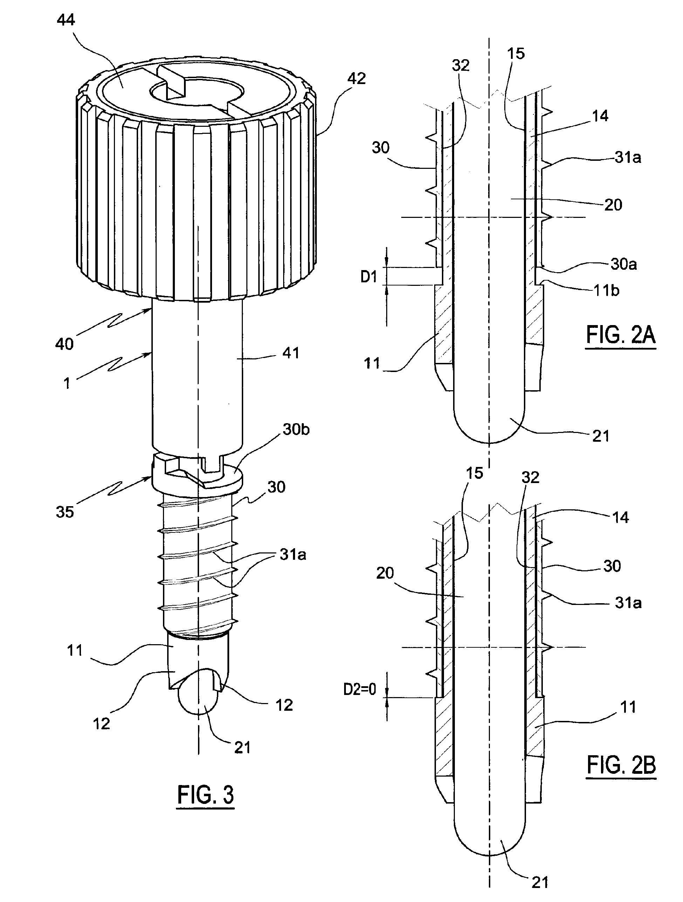

In the embodiment shown in figures 1 to 7, these means are determined by the length of the free axial portion of the rod 14 on which the tubular element 30 can slide, in relation to the length of the tubular element 30. The milling element 10 is free to move axially relative to the tubular element 30 between a *fully advanced position*, in which the milling head 11 projects forward the maximum possible distance D1 from the tubular element 30 (see figures 1, 2, and 2A), and a *fully withdrawn position*, in which the milling head 11 is at the minimum possible distance D2 from the tubular element 30, in particular it is in contact there-with (D2 equal to zero) (see figure 2B).

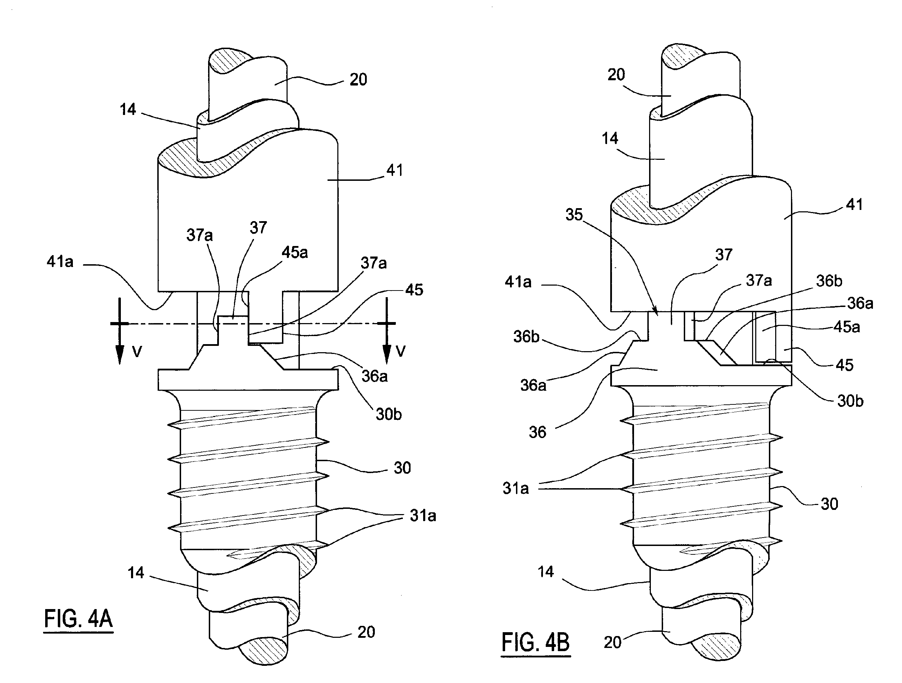

In particular, the above-mentioned profiled raised portions 35 and 45 are conformed such that they maintain the tubular element 30 at a predefined maximum axial distance (which substantially corresponds to the above-mentioned *fully withdrawn position* of the milling element 10) relative to the drive element 40, when the elements are positioned in reciprocal torsional contact (the position of figure 4A), or to enable an approach of the two elements 30 and 40 to a minimal axial distance (which corresponds to the above-mentioned *fully advanced position* of the milling element 10) following angular displacement relative to the reciprocal torsional contact position (the position in figure 4B).

In detail, the profiled raised portion 35, joined to the tubular element 30, has a widened base 36 with two surfaces 36a inclined and converging, and a central raised straight portion 37 with two parallel sides of axial development 37a that project axially relative to the base 36. The other raised portion 45, joined to the drive element 40, has a raised central portion with two parallel sides of axial development 45a (Figs. 4A, 4B). Alternatively, the raised portion joined to the threaded tubular element 30 can have the shape described herein for the raised portion united with the drive element 40 and vice-versa.

Rotating the drive element 40 relative to the tubular element 30, the raised portion 45 first comes into contact with the base 36 of the relief portion 35, then slides along one of the inclined surfaces 36a; following this sliding, as it approaches the central raised portion 37, the two elements 30 and 40 move away from each other axially. When the axial sides 37a and 45a then come into reciprocal contact (figure 4A), the tubular element 30 and the drive element 40, are situated at a predefined distance (corresponding to the fully withdrawn position of the milling head 11), without the possibility of moving closer, due also to the fact that at the base of the central raised portion 37 two sections 36b are located on transversal planes (at 90 degrees relative to the axial direction) serving as end stops against the highest extremity of the raised portion 45, preventing its approach to the tubular element 30.

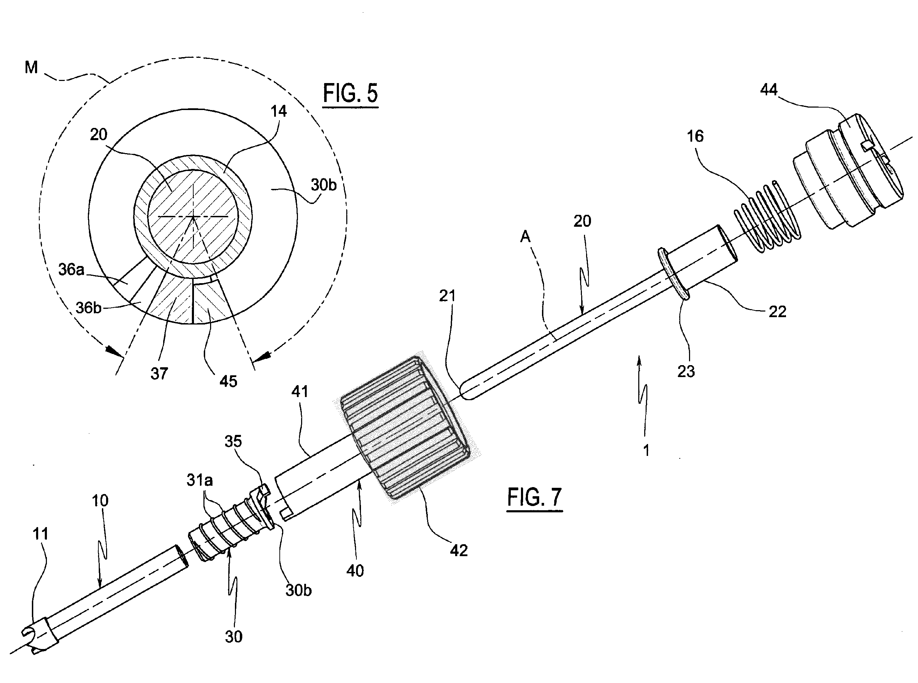

By rotating the drive element 40 relative to the tubular element 30 in the opposite direction so as to distance the two relief portions 35 and 45 from each other, the rotary movement of the element 40 is independent relative to element 30, up to an angle M (figure 5) less than 360 degrees; further, these elements 30 and 40 can be axially moved towards each other until the upper apex of the relief portion 37 stops in contact with the edge 40a of the element 40 (or the upper apex of the relief portion 45 stops in contact with the edge 30b of element 30), which determines the minimum distance between elements 30 and 40 (Figure 4B).

The axial through-hole 15, extends with a coaxial hole 46 of substantially the same diameter and passing through the drive element 40; the probe device 20, of matching diameter, is located inside the holes 15 and 46, the probe device 20 being subject to the action of elements acting to push it axially so that the forward portion 21 thereof projects with respect to the forward portion of the milling element.

The probe element 20 has a rear portion 22 which is visible to the operator.

In particular, the rear portion 22 of the probe element is designed to be visible at the rear of the handle defined by the rear portion 42 of the drive element 40.

The probe element 20 can be moved axially relative to the milling element 10 and relative to the handle 42.

In particular, this displacement is produced by pressure applied to the rear portion 22, such as to cause the forward portion 21 to project for a predefined distance beyond the forward portion of the milling element 10.

In the embodiment shown in the figures, the rear portion 22 of the probe element 20 has a stop surface 23a for axial movement thereof towards the forward end and it is subject to the axial thrust pressure of a precompressed helical spring 16 acting to push the probe element 20 forward. In particular the portion 22 has a greater diameter than the axial hole 46, thus forming the stop surface 23a.

In particular, the rear portion 22 is housed in a cylindrical concavity 43 formed in the rear portion 42, which is coaxial with axis A and faces rearwards; the spring 16 is located inside the concavity 43 and is compressed between a radial raised portion 23 formed in the rear portion 22 and a stop element 44 coupled helically to the concavity 43, with the possibility of adjusting the axial position of the spring 16 and thus its level of precompression, and consequently the axial pressure acting on the probe element 20.

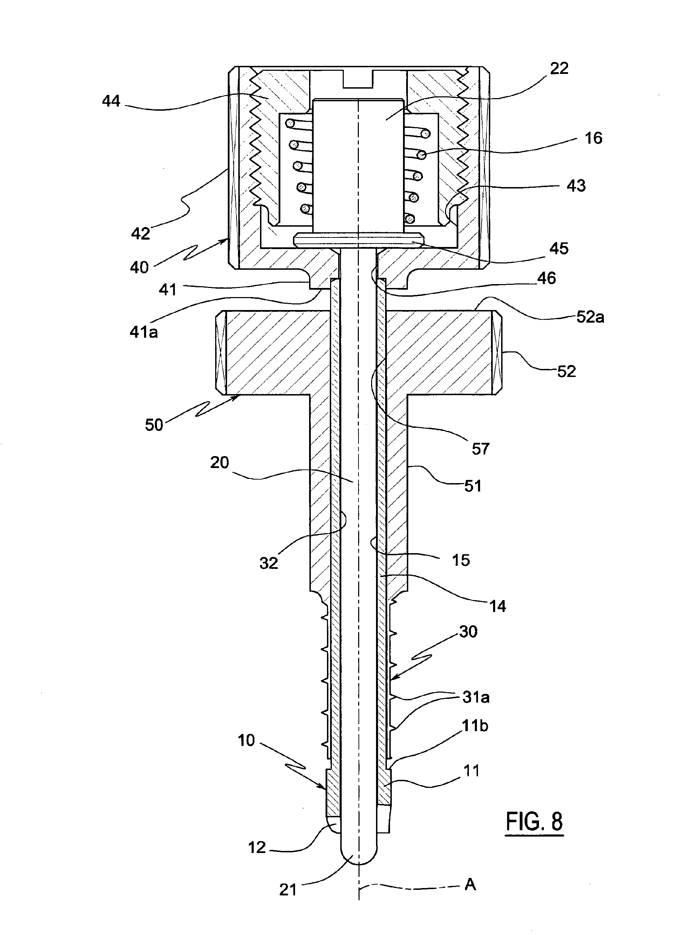

Figure 8 shows a second embodiment of the invention.

The second embodiment differs from the first principally due to the fact that the drive element 40 rotates only the milling element 10 and not also the tubular element 30; consequently the described profiled raised portions 35 and 45 are absent and the drive element 40 can rotate freely relative to the tubular element 30.

The tubular element 30 is instead rotated by a dedicated second drive element 50, independent of the first drive element 40.

In particular the drive element 50 is located forward of the drive element 40 and it is coaxially and solidly fixed to the rear end portion of the tubular element 30; the rod 14 of the milling element 10 passes through a matching axial through-hole 57 formed in the drive element 50.

The second drive element 50 comprises a tubular shank 51 fixed coaxially and solidly to the rear end portion of the tubular element 30 and a rear portion 52, of greater diameter relative to the shank 52, shaped as a circular handle serving to enable the operator to manually activate the milling element 10, with their fingers, in rotation and in axial translation. Preferably, the rear portion 52 has a diameter that is greater than the corresponding portion 42.

The two drive elements 40 and 50 are free to rotate reciprocally, even if they are in contact.

This embodiment also includes elements acting to limit the axial movement of the milling element 10 relative to the tubular element 30 to a predetermined extent.

In particular, the milling element 10, together with the drive element 40, is free to move axially relative to the tubular element 30 and the second drive element 50, between a *fully advanced position* (figure 2A), in which the milling head 11 projects forward the maximum possible distance D1 from the tubular element 30, and a *fully withdrawn position* (figure 2B), in which the milling element 11 is at the minimum possible distance D2 from the tubular element 30, and in particular is in contact there-with (D2 equal to zero).

The axial displacement between the elements 10 and 30 is delimited between the contact of a forward edge 30a of the tubular element 30 with a rear edge 11 b of the milling head 11 (which determines the *fully advanced position*) (Figure 2A) and the contact of a transversal rear surface 52b of the rear portion 52 with a forward circular protrusion 42a of the shank 42 (which determines the *fully withdrawn position*) (figure 2B). There follows a description of use of the instrument.

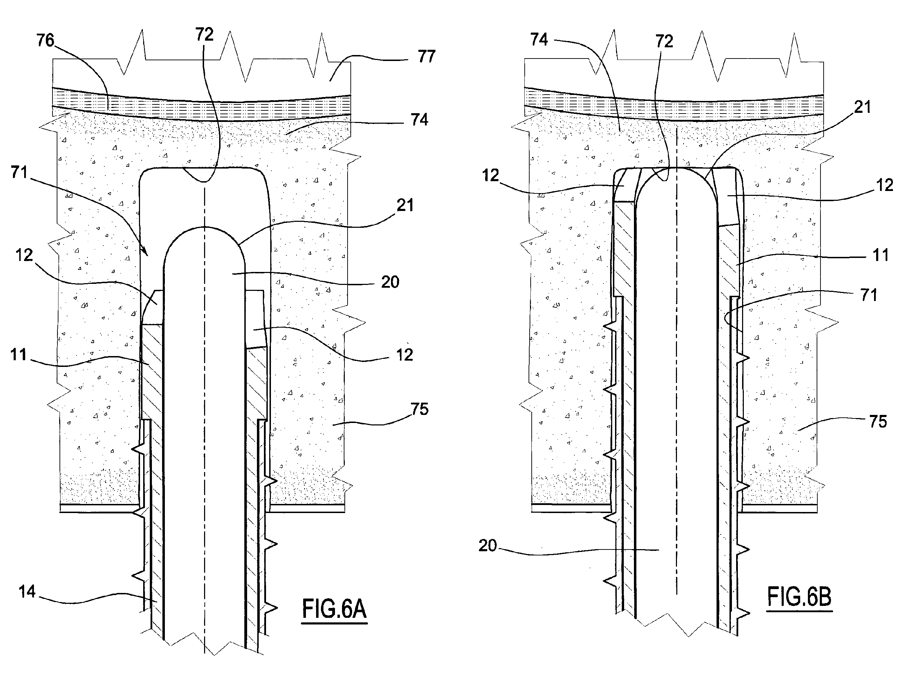

A typical use of the instrument illustrated is to extend the final part of an initially blind hole, realized previously in a jaw bone. Figure 6A shows the preliminary blind section 71 of the hole, which is realised in the jaw bone 75 in a known way (for example, using a motorized milling instrument, possibly with the use of radiographic viewing means), the end surface 72 of which lies at a relatively short, though safe, distance (a few millimetres) from the Schneider membrane 76 located at the bottom of the maxillary sinus 77.

The instrument 1 is suitable for extending the final part of the hole 71 up until it breaks or destroys the thin upper wall 74 of bone cortex which separates the end surface 72 of the hole 71 from the membrane 76, without causing harmful lesions to the membrane.

For this purpose, first (figure 6A), the tubular element 30 is rotated inside the hole 71, such that the helical thread 31 a thereof engages with the cylindrical surface of the hole 71, into which the thread penetrates thanks to the sharp edges thereof. At this stage the milling head 11 is in the above-mentioned *withdrawn position* relative to the tubular element 30 (in particular, they are in contact).

In a first phase (advancement phase), by rotating the element 30 the penetration of the forward end of the instrument 1 (the milling head 11 and the forward portion of the tubular element 30) is induced inside the hole 71. The instrument is made to advance axially in the hole until the forward portion of the milling head 11 comes into contact with the end surface 72 (figure 6B). At this point the forward portion 21 of the probe 20 is stationary against the surface 72, aligned with the forward portion of the milling head 11; the rear portion 22 of the probe 20, is now in a position relative to the rear portion of the drive element 40 which can be used as an initial reference position.

The point at which the milling head 11 comes into contact with the end surface 72 is actually perceived by the operator, especially because the probe element 20 is pushed back into the hole 15 such that the rear portion 22 thereof moves back into the reference position and this movement is seen by the operator, and the advance of the instrument is halted. Furthermore, the operator notices an increased resistance to rotation of the tubular element 30 together with the fact that the milling head 11 is axially locked in the above-mentioned *withdrawn position* and cannot perform forward axial movement.

In the first embodiment of the instrument, the rotation of the tubular element 30 is produced by manually rotating the drive element 40, which, through the reciprocally engaging elements 35 and 45, transmits torque to the tubular element 30.

In the second embodiment of the instrument, the rotation of the tubular element 30 is produced by manually rotating the drive element 50, which drives only the element 30, and the milling head 11 is not set in rotation.

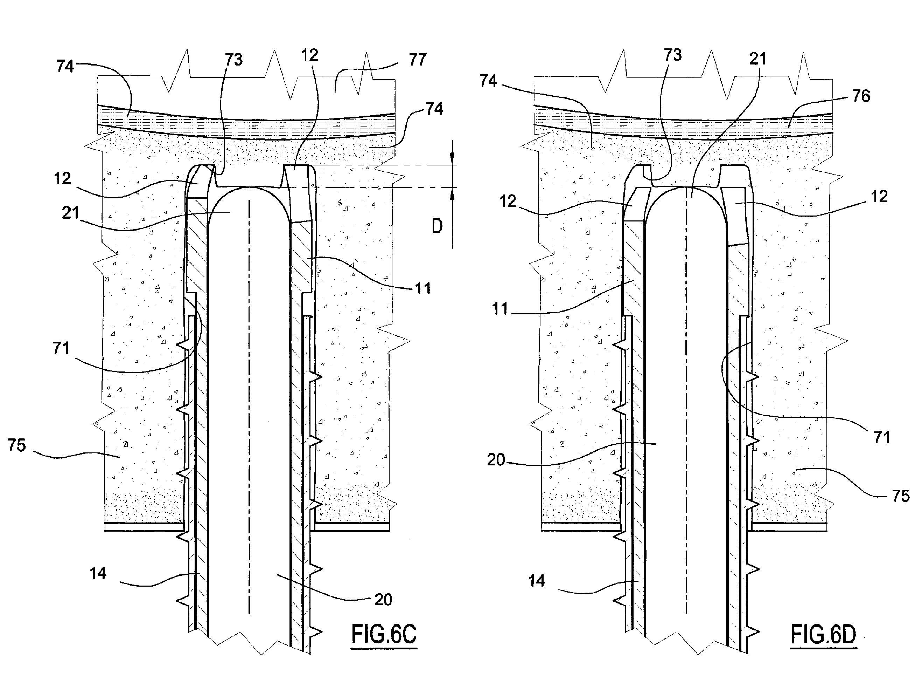

In the second phase (the milling phase), only the milling element 10 is rotated, and it is also pushed forward relative to the tubular element 30, in order to create on the end of the hole 71, by way of the action of the raised milling ridges 12, a groove 73 in the form of a circular channel surrounding a circular area 72a of the end surface 72, facing the hole 15, which remains unaffected by the action of the milling ridges 12 (see figure 6C).

In the first embodiment, operation is manual using the drive element 40, the raised portion 45 is distanced from the raised portion 35, and the milling element 10 is rotated relative to the tubular element 30, with alternating rotation by an angle included within the above-mentioned angular range M which does not interfere with the raised portion 35. At the same time the milling head 11 is pushed forward manually so that the combined actions (axial pressure and rotation) cause removal of bone tissue at the point of contact of the raised milling ridges 12.

In the second embodiment, operation is manual using the drive element 50 on the milling head 11, rotating it and at the same time pushing it manually forward such that the combined actions (axial pressure and rotation) cause the removal of bone tissue at the point of contact of the raised milling ridges 12.

The milling action continues at most until the milling head 11 reaches the maximum advanced position, this being to a depth equal to the difference D between the maximum distance D1 and the minimum distance D2. Consequently, a peripheral circular groove 73 is created on the end surface 72 with an axial depth of maximum depth D, which develops around the circumference of the end surface 72, axially approaching the membrane 76.

In the subsequent phase (third phase, advancement), the tubular element 30 is further rotated in order to cause a further axial advance of the instrument proportional to the imposed rotation.

In particular, in the first embodiment, the tubular element 30 is again rotated manually, using the drive element 40 in order to act on the tubular element 30 through the reciprocally engaging elements 35 and 45, causing a further axial advance of the element 30 proportional to the imposed rotation. In this phase, following the approach into contact of the raised portions 35 and 45 the milling element 10 is first returned into the fully withdrawn position in relation to the tubular element 30, and its raised milling ridges 12 are withdrawn from the groove 73 (see figure 6D) so that, while the tubular element 30 rotates and screws into the hole 71, the milling ridges 12 do not mill the bone.

Alternatively the rotation of the tubular element 30 is imposed by the drive element 50 (in the second embodiment) which acts directly on the tubular element 30; in this case the milling head 11 does not rotate and the raised milling ridges 12 do not mill the bone.

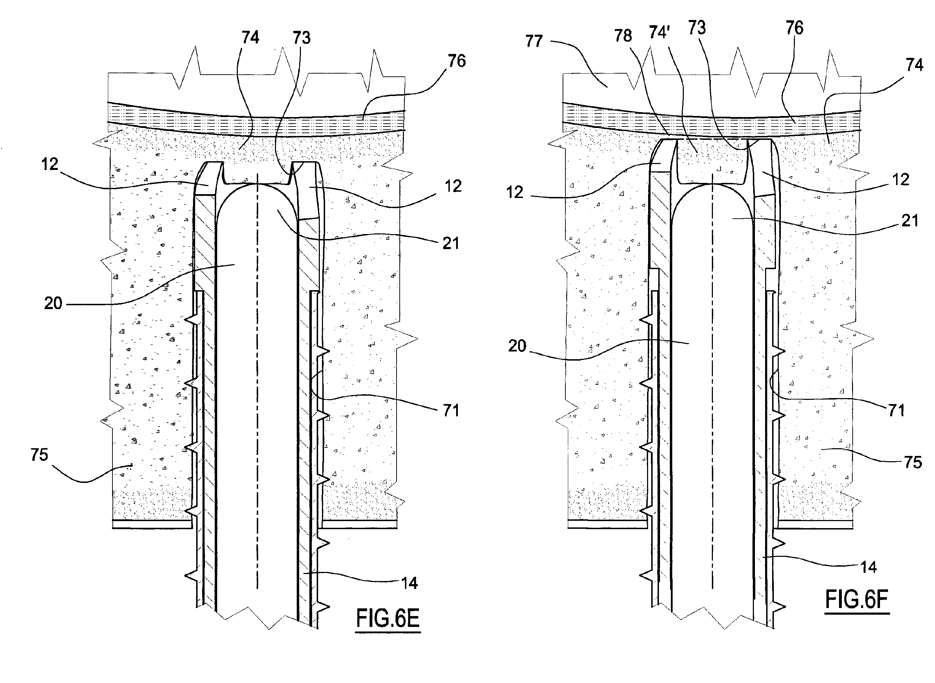

When the milling head 11 comes into contact with the end surface of the peripheral groove (see figure 6E), this is perceived by the operator, both due to the increased resistance of the tubular element 30 and because the drive element 40 can no longer slide axially. The operator also perceives that the rear portion of the probe has moved by a measurable distance in relation to the initial reference position. At this point the penetration of the tubular element 30 is stopped.

In the subsequent phase (fourth phase, milling) only the milling element 10 is rotated in order to increase by a further axial distance the depth of the circular groove 72 created during the previous phase (Figure 6F). In practice, the milling element 10 is operated in the same way as described above for the second phase.

It is possible to proceed with further cycles of advance of the tubular element 30 and subsequent milling as described above, progressively increasing the depth of the groove 73 until a residual portion 74' of the wall 74 is defined, in the form of a small disk delimitated by the groove, remaining attached to the jaw bone 75 by a residual layer 78 of relatively very thin bone (see figure 6F).

In order to finally detach this residual portion 74', which closes the hole 71, from the bone 75, a predetermined axial pressure can be applied to the probe element 20. This pressure can be produced by the spring 16 which automatically breaks the layer 78, detaches the portion, and opens the hole 71 towards the membrane 76; the latter is not damaged because the probe element 20 is designed with a rounded tip.

Alternatively, it can be arranged that the axial pressure on the probe element 20 be applied manually by the operator, who, for example, pushes with a finger on the rear portion of the probe element 20 which extends outside of the drive element 40.

Alternatively, when the residual portion of bone wall 74 is sufficiently weakened, the tubular element 30 can be rotated so that the milling head 11 is made to advance axially against the end surface until the residual portion 74 is detached.

In a further alternative embodiment (not shown in the figures), the drive element 40 is activated mechanically in rotation, for example by drive transmission elements equipped to the rear part of the same.

A similar mechanical drive solution can also be applied to the second drive element 50 of the second embodiment described above.

The invention makes it possible to perform axial milling of a bone hole while maintaining control of the position of the device relative to the hole. The instrument 1 enables the operator to perceive, by way of the resistance encountered during axial advance, by screwing, along the hole 71, possibly also through the action of the probe 20, the point at which the milling head 11 reaches the end 72 of the previously formed hole 71, or the end of the cavity produced by the milling head 11. Furthermore, each phase in which the milling element 10 is rotated while the tubular element 30 is stationary, produces a groove 73, the depth of which has a constant predefined value; in particular, equal to the value (D1 - D2) of the axial translation completed by the head 11 passing from the maximum or full withdrawn position to the maximum or full forward position.

As the operator can perceive the point which the advancement phase has reached (the milling head 11 reaches the end of the previously formed hole 71, or the end of the cavity produced by it) and knows the depth of the milling phase (the cavity realized by the milling head 11), the depth of the cavity being created can be constantly monitored.

Further, the operations of further axial advancement of the milling head 11 can be checked with the probe element 20; in particular, while the milling element 10 is made to advance further, to excavate a further axial section of cavity, the probe element 20 remains axially stationary against the central area of the end surface 72 (which is not subjected to milling). Consequently, since the probe element 20 is designed to have millimetre markings, it becomes a means for constant control and for determining the measured position of the milling head 11 relative to the position of the end 72 of the bone hole.

Further, by way of an axial pressure applied to the probe element 20, detachment of the residual portion 74' of the bone wall can be achieved as soon as the residual portion 74' has reached a degree of breaking resistance which is less than the force applied to the probe element 20.

In particular, it can be arranged so that this detachment occurs before the raised cutting ridges 12 have passed beyond, even at a single point, the thickness of the wall 74; the detachment thus occurs without the cutting ridges being able to come into contact with the delicate membrane 76. Furthermore, since the penetration of the tubular element 30 along the axis of the hole 71 is directly dependent on the angle of rotation of the same, by way of the pitch of the thread 31 a, through the control of the rotation applied to the tubular element 30, it is possible to maintain control of the extent of axial penetration of the tubular element 30, and thus of the forward end of the instrument, in the hole 71.

Obviously numerous modifications of a practical-technical nature might be brought to the invention without its forsaking the ambit of the inventive idea as claimed below in the claims.

## Claims

1. A surgical bone milling instrument, comprising a milling element (10) having a longitudinal axis (A) and a forward portion designed to mill bone, the milling element (10) rotating around the longitudinal axis (A) thereof, comprising a probe element (20) of elongate shape located internally and coaxially of the milling element (10) and being longitudinally slideable through the milling element (10), with a forward portion thereof projecting in relation to the forward portion of the milling element (10), **characterized in that** it comprises a tubular element (30) coaxially passed through by the milling element (10), the milling head (11) thereof being located forward of the tubular element (30), the tubular element (30) having an external surface with a circular cross-section provided with a thread destined to engage, by helical coupling, in a hole formed in bone.

2. The instrument of claim 1, **characterized in that** it comprises pushing elements which axially push the probe element (20), in order to cause the forward portion of the probe element (20) to project beyond the forward portion of the milling element (10).

3. The instrument of claim 2, **characterized in that** it comprises means for adjusting an axial pressure applied to the probe element (20).

4. The instrument of claim 1, **characterized in that** it comprises means for signalling displacements of the probe element (20) relative to the milling element (10).

5. The instrument of claim 4, **characterized in that** the probe element (20) has a rear portion which is visible to the operator.

6. The instrument of claim 1, comprising a handle (42, 52) for manipulating the instrument, **characterized in that** the rear portion of the probe element (20) is designed to remain visible at a rear of the handle (42, 52).

7. The instrument of claim 6, **characterized in that** the probe element (20) is designed to be moved axially by way of pressure applied to its rear portion, causing the forward portion thereof to project by a predefined distance beyond a front end of the milling element (10).

8. The instrument of claim 1, **characterized in that** the probe element (20) is equipped with millimetric markings for constant control and determination of a measured position of the milling head (11) in relation to a position of an end (72) of the bone hole.

9. The instrument of claim 8, **characterized in that** the milling element (10) is associated rotatably about the longitudinal axis thereof to the tubular element (30), and can translate axially with respect to the tubular element (30).

10. The instrument of claim 9, **characterized in that** it comprises a drive element (40), located rear of the tubular element (30), which drive element (40) is solidly constrained to the milling element (10), rotates and displaces the milling element (10) axially in relation to the tubular element (30).

11. The instrument of claim 9, **characterized in that** it comprises means for limiting, to a predetermined extent, the axial displacement of the milling element (10) in relation to the tubular element (30).

12. The instrument of claim 10, **characterized in that** the drive element (40) transmits torsional torque to the tubular element (30).

13. The instrument of claim 12, **characterized in that** the drive element (40) is free to slide axially and rotate relative to the tubular element (30) and is torsionally engaged with the tubular element (30) via means for reciprocally engaging which leave the drive element (40) free to be rotated by an angle of less than 360 degrees, and to be displaced axially relative to the tubular element (30).

14. The instrument of claim 13, **characterized in that** the means for reciprocally engaging comprise profiled raised portions (35, 45) protruding in an axial direction from the tubular element (30) and respectively from the drive element (40), which are designed to come into reciprocal contact following reciprocal rotation in order to transmit a torsional torque drive.

15. The instrument of claim 14, **characterized in that** the profiled raised portions (35) and (45) are shaped such as to maintain the tubular element (30) and the drive element (40) at a predetermined maximum axial distance when the tubular element (30) and the drive element (40) are positioned in reciprocal torsional contact, and to permit a nearing to a minimum axial distance of the tubular element (30) and the drive element (40) by angular displacement thereof with respect to the position of reciprocal torsional contact.

16. The instrument of claim 10, **characterized in that** it comprises a second drive element (50) fixed to the tubular element (30) and rotating the tubular element (30).

17. The instrument of claim 16, **characterized in that** the second drive element (50) is coaxial to, and located forward of, the first drive element (40).

## Patentansprüche

1. Chirurgisches Knochenfräsinstrument, umfassend ein Fräselement (10) mit einer Längsachse (A) und einem vorderen Abschnitt, der dafür gestaltet ist, Knochen zu fräsen, wobei sich das Fräselement (10) um seine Längsachse (A) dreht und ein Sondenelement (20) mit länglicher Form umfasst, das im und koaxial zum Fräselement (10) angeordnet und in Längsrichtung durch das Fräselement (10) gleitfähig ist, wobei dessen vorderer Abschnitt im Verhältnis zum vorderen Abschnitt des Fräselements (10) hervorsteht, **dadurch gekennzeichnet, dass** es ein röhrenförmiges Element (30) umfasst, das koaxial durch das Fräselement (10) verläuft, wobei dessen Fräskopf (11) vor dem röhrenförmigen Element (30) angeordnet ist, das röhrenförmige Element (30) eine Außenfläche mit einem kreisrunden Querschnitt aufweist, die mit einem Gewinde versehen ist, das dafür vorgesehen ist, durch spiralförmiges Koppeln in eine in einem Knochen gebildete Öffnung einzugreifen.

2. Instrument nach Anspruch 1, **dadurch gekennzeichnet, dass** es Drückelemente umfasst, die das Sondenelement (20) axial vorwärts drücken, um zu bewirken, dass der vordere Abschnitt des Sondenelements (20) über den vorderen Abschnitt des Fräselements (10) hervorsteht.

3. Instrument nach Anspruch 2, **dadurch gekennzeichnet, dass** es Mittel zum Justieren eines Axialdrucks umfasst, der auf das Sondenelement (20) ausgeübt wird.

4. Instrument nach Anspruch 1, **dadurch gekennzeichnet, dass** es Mittel zum Signalisieren von Verlagerungen des Sondenelements (20) im Verhältnis zum Fräselement (10) umfasst.

5. Instrument nach Anspruch 4, **dadurch gekennzeichnet, dass** das Sondenelement (20) einen hinteren Abschnitt aufweist, der für den Operateur sichtbar ist.

6. Instrument nach Anspruch 1, einen Griff (42, 52) zum Handhaben des Instruments umfassend, **dadurch gekennzeichnet, dass** der hintere Abschnitt des Sondenelements (20) dafür gestaltet ist, an der Rückseite des Griffs (42, 52) sichtbar zu bleiben.

7. Instrument nach Anspruch 6, **dadurch gekennzeichnet, dass** das Sondenelement (20) dafür gestaltet ist, mit Hilfe von Druck, der auf dessen hinteren Abschnitt angelegt wird, axial bewegt zu werden, was bewirkt, dass der vordere Abschnitt des Sondenelements um einen vordefinierten Abstand über ein vorderes Ende des Fräselements (10) hinausragt.

8. Instrument nach Anspruch 1, **dadurch gekennzeichnet, dass** das Sondenelement (20) mit Millimetermarkierungen für die ständige Kontrolle und das Bestimmen einer Messposition des Fräskopfes (11) im Verhältnis zu einer Position eines Endes (72) der Knochenöffnung versehen ist.

9. Instrument nach Anspruch 8, **dadurch gekennzeichnet, dass** das Fräselement (10) um seine Längsachse drehbar dem röhrenförmigen Element (30) zugeordnet ist und im Verhältnis zum röhrenförmigen Element (30) axial verlagert werden kann.

10. Instrument nach Anspruch 9, **dadurch gekennzeichnet, dass** es ein Antriebselement (40) umfasst, das hinter dem röhrenförmigen Element (30) angeordnet ist, wobei das Antriebselement (40) fest am Fräselement (10) verspannt ist, das Fräselement (10) dreht und im Verhältnis zum röhrenförmigen Element (30) axial verlagert.

11. Instrument nach Anspruch 9, **dadurch gekennzeichnet, dass** es Mittel zum Begrenzen der axialen Verlagerung des Fräselements (10) im Verhältnis zum röhrenförmigen Element (30) in einem festgelegten Umfang umfasst.

12. Instrument nach Anspruch 10, **dadurch gekennzeichnet, dass** das Antriebselement (40) ein Torsionsmoment auf das röhrenförmige Element (30) überträgt.

13. Instrument nach Anspruch 12, **dadurch gekennzeichnet, dass** das Antriebselement (40) im Verhältnis zum röhrenförmigen Element (30) frei dreht und axial gleitet und über Mittel zum gegenseitigen Eingriff, die das Antriebselement (40) in einem Winkel von weniger als 360° frei drehen lassen und es im Verhältnis zum röhrenförmigen Element (30) frei axial verlagern lassen, drehsteif mit dem röhrenförmigen Element (30) in Eingriff steht.

14. Instrument nach Anspruch 13, **dadurch gekennzeichnet, dass** die Mittel zum gegenseitigen Eingriff profilierte erhöhte Abschnitte (35, 45) umfassen, die in axialer Richtung aus dem röhrenförmigen Element (30) beziehungsweise dem Antriebselement (40) hervorragen, die dafür gestaltet sind, auf die gegenseitige Drehung hin in gegenseitigen Kontakt zu kommen, um einen Torsionsmomentantrieb zu übertragen.

15. Instrument nach Anspruch 14, **dadurch gekennzeichnet, dass** die profilierten erhöhten Abschnitte (35, 45) derart geformt sind, dass das röhrenförmige Element (30) und das Antriebselement (40) in einem festgelegten maximalen axialen Abstand bleiben, wenn das röhrenförmige Element (30) und das Antriebselement (40) in gegenseitigem Drehkontakt stehen, und ein Annähern an einen minimalen axialen Abstand zwischen dem röhrenförmigen Element und dem Antriebselement (40) durch Winkelverlagerung des Antriebselements im Verhältnis zur Position des gegenseitigen Drehkontakts gestatten.

16. Instrument nach Anspruch 10, **dadurch gekennzeichnet, dass** es ein zweites Antriebselement (50) umfasst, das am röhrenförmigen Element (30) befestigt ist und das röhrenförmige Element (30) dreht.

17. Instrument nach Anspruch 16, **dadurch gekennzeichnet, dass** das zweite Antriebselement (50) vor dem ersten Antriebselement (40) und koaxial zu diesem angeordnet ist.

## Revendications

1. Instrument chirurgical de fraisage des os, comprenant un élément de fraisage (10) ayant un axe longitudinal (A) et une portion avant conçue pour fraiser de l'os, l'élément de fraisage (10) tournant autour de son axe longitudinal (A), comprenant un élément de sonde (20) de forme allongée situé à l'intérieur de l'élément de fraisage (10) et coaxialement par rapport à celui-ci et pouvant glisser longitudinalement à travers l'élément de fraisage (10), dont une portion avant ressort par rapport à la portion avant de l'élément de fraisage (10), **caractérisé en ce qu'**il comprend un élément tubulaire (30) traversé coaxialement par l'élément de fraisage (10), sa tête de fraisage (11) étant située devant l'élément tubulaire (30), l'élément tubulaire (30) ayant une surface externe avec une section transversale circulaire pourvue d'un filetage destiné à s'engager par accouplement hélicoïdal dans un trou formé dans l'os.

2. Instrument selon la revendication 1, **caractérisé en ce qu'**il comprend des éléments de poussée qui poussent l'élément de sonde (20) axialement, de manière à forcer la portion avant de l'élément de sonde (20) à ressortir au-delà de la portion avant de l'élément de fraisage (10).

3. Instrument selon la revendication 2, **caractérisé en ce qu'**il comprend un moyen pour ajuster une pression axiale appliquée à l'élément de sonde (20).

4. Instrument selon la revendication 1, caractérisé en qu'il comprend un moyen de signalisation de déplacements de l'élément de sonde (20) par rapport à l'élément de fraisage (10).

5. Instrument selon la revendication 4, **caractérisé en ce que** l'élément de sonde (20) comporte une portion arrière qui est visible pour l'opérateur.

6. Instrument selon la revendication 1, comprenant une poignée (42, 52) de manipulation de l'instrument, **caractérisé en ce que** la portion arrière de l'élément de sonde (20) est conçue pour rester visible à l'arrière de la poignée (42, 52).

7. Instrument selon la revendication 6, **caractérisé en ce que** l'élément de sonde (20) est conçu pour être déplacé axialement par une pression appliquée à sa portion arrière, de manière à forcer sa portion avant à ressortir d'une distance prédéfinie au-delà d'une extrémité avant de l'élément de fraisage (10).

8. Instrument selon la revendication 1, **caractérisé en ce que** l'élément de sonde (20) est pourvu de marquages millimétriques pour le contrôle constant et la détermination d'une position mesurée de la tête de fraisage (11) par rapport à la position d'une extrémité (72) du trou dans l'os.

9. Instrument selon la revendication 8, **caractérisé en ce que** l'élément de fraisage (10) est connecté à l'élément tubulaire (30) de manière rotative autour de son axe longitudinal, et peut se déplacer axialement en translation par rapport à l'élément tubulaire (30).

10. Instrument selon la revendication 9, **caractérisé en ce qu'**il comprend un élément d'entraînement (40), situé derrière l'élément tubulaire (30), ledit élément d'entraînement (40) étant contraint de manière solidaire à l'élément de fraisage (10), tournant et déplaçant l'élément de fraisage (10) axialement par rapport à l'élément tubulaire (30).

11. Instrument selon la revendication 9, **caractérisé en ce qu'**il comprend un moyen de limitation, dans une plage prédéterminée, du déplacement axial de l'élément de fraisage (10) par rapport à l'élément tubulaire (30).

12. Instrument selon la revendication 10, **caractérisé en ce que** l'élément d'entraînement (40) transmet un couple de torsion à l'élément tubulaire (30).

13. Instrument selon la revendication 12, **caractérisé en ce que** l'élément d'entraînement (40) peut librement glisser axialement et tourner par rapport à l'élément tubulaire (30) et est engagé en torsion avec l'élément tubulaire (30) via un moyen d'engagement réciproque qui procure à l'élément d'entraînement (40) la liberté de pivoter d'un angle inférieur à 360 degrés et de se déplacer axialement par rapport à l'élément tubulaire (30).

14. Instrument selon la revendication 13, **caractérisé en ce que** le moyen d'engagement réciproque comprend des portions profilées relevées (35, 45) qui ressortent en direction axiale respectivement de l'élément tubulaire (30) et de l'élément d'entraînement (40), qui sont conçues pour entrer en contact réciproque suite à une rotation réciproque de manière à transmettre un couple de torsion d'entraînement.

15. Instrument selon la revendication 14, **caractérisé en ce que** les portions profilées relevées (35) et (45) sont conformées de manière à maintenir l'élément tubulaire (30) et l'élément d'entraînement (40) à une distance axiale maximale prédéterminée quand l'élément tubulaire (30) et l'élément d'entraînement (40) sont positionnés en contact torsionnel réciproque, et pour permettre un rapprochement à une distance axiale minimale de l'élément tubulaire (30) et de l'élément d'entraînement (40) grâce à leur déplacement angulaire par rapport à la position de contact torsionnel réciproque.

16. Instrument selon la revendication 10, **caractérisé en ce qu'**il comprend un deuxième élément d'entraînement (50) fixé à l'élément tubulaire (30) et entraînant l'élément tubulaire (30) en rotation.

17. Instrument selon la revendication 16, **caractérisé en ce que** le deuxième élément d'entraînement (50) est coaxial avec le premier élément d'entraînement (40) et situé devant celui-ci.
